# EUROPÄISCHE PATENTANMELDUNG

(11) **EP 1 707 642 A2**
(43) Veröffentlichungstag der Anmeldung: **04.10.2006**
(21) Anmeldenummer: 06090098.2
(22) Anmeldetag: 19.06.2001
(51) Int. Cl.: C12Q 1/68

(54) **Verfahren zum Nachweis von Cytosin-Methylierungen**

(30) Priorität: 19.06.2000 DE 10029915
(62) Teilanmeldung aus: 01953113.6
(71) Anmelder: Epigenomics AG, 10178 Berlin (DE)
(72) Erfinder: Berlin, Kurt, Dr., 14532 Stahnsdorf (DE)
(74) Vertreter: Schubert, Klemens

(57) **Zusammenfassung**

Beschrieben wird ein Verfahren zum Nachweis von 5-Methylcytosin in genomischen DNA-Proben. Zuerst wird eine genomische DNA aus einer DNA-Probe mit einem Reagenz chemisch umgesetzt, wobei 5-Methylcytosin und Cytosin unterschiedlich reagieren. Anschließend wird die vorbehandelte DNA unter Verwendung einer Polymerase mit Primern unterschiedlicher Sequenz amplifiziert. Im nächsten Schritt wird die amplifizierte genomische DNA auf einen Oligonukleotid Array hybridisiert und PCR-Produkte erhalten, die mit einer Markierung versehen sein müssen. Alternativ können die PCR-Produkte in einer Primer Extention Reaktion verlängert werden, wobei auch die Verlängerungsprodukte mit einer Markierung versehen sind.

Im letzten Schritt werden die verlängerten Oligonukleotide auf das Vorhandensein der Markierung untersucht.

## Beschreibung

Die vorliegende Erfindung betrifft ein Verfahren zum Nachweis von Cytosin-Methylierungen in DNA.

Die nach den methodischen Entwicklungen der letzten Jahre in der Molekularbiologie gut studierten Beobachtungsebenen sind die Gene selbst, die Übersetzung dieser Gene in RNA und die daraus entstehenden Proteine. Wann im Laufe der Entwicklung eines Individuums welches Gen angeschaltet wird und wie Aktivieren und Inhibieren bestimmter Gene in bestimmten Zellen und Geweben gesteuert wird, ist mit Ausmaß und Charakter der Methylierung der Gene bzw. des Genoms korrelierbar. Insofern äußern sich pathogene Zustände in einem veränderten Methylierungsmuster einzelner Gene oder des Genoms.

5-Methylcytosin ist die häufigste kovalent modifizierte Base in der DNA eukaryotischer Zellen. Sie spielt beispielsweise eine Rolle in der Regulation der Transkription, beim genetischen Imprinting und in der Tumorgenese. Die Identifizierung von 5-Methylcytosin als Bestandteil genetischer Information ist daher von erheblichem Interesse. 5-Methylcytosin-Positionen können jedoch nicht durch Sequenzierung identifiziert werden, da 5-Methylcytosin das gleiche Basenpaarungsverhalten aufweist wie Cytosin. Darüber hinaus geht bei einer PCR-Amplifikation die epigenetische Information, welche die 5-Methylcytosine tragen, vollständig verloren.

Eine relativ neue und die inzwischen am häufigsten angewandte Methode zur Untersuchung von DNA auf 5-Methylcytosin beruht auf der spezifischen Reaktion von Bisulfit mit Cytosin, das nach anschließender alkalischer Hydrolyse in Uracil umgewandelt wird, welches in seinem Basenpaarungsverhalten dem Thymidin entspricht. 5-Methylcytosin wird dagegen unter diesen Bedingungen nicht modifiziert. Damit wird die ursprüngliche DNA so umgewandelt, dass Methylcytosin, welches ursprünglich durch sein Hybridisierungsverhalten vom Cytosin nicht unterschieden werden kann, jetzt durch "normale" molekularbiologische Techniken als einzig verbliebenes Cytosin beispielsweise durch Amplifikation und Hybridisierung oder Sequenzierung nachgewiesen werden kann. Alle diese Techniken beruhen auf Basenpaarung, welche jetzt voll ausgenutzt wird. Der Stand der Technik, was die Empfindlichkeit betrifft, wird durch ein Verfahren definiert, welches die zu untersuchende DNA in einer Agarose-Matrix einschließt, dadurch die Diffusion und Renaturierung der DNA (Bisulfit reagiert nur an einzelsträngiger DNA) verhindert und alle Fällungs- und Reinigungsschritte durch schnelle Dialyse ersetzt (Olek, A. et al., Nucl. Acids. Res. 1996, 24, 5064-5066). Mit dieser Methode können einzelne Zellen untersucht werden, was das Potential der Methode veranschaulicht. Allerdings werden bisher nur einzelne Regionen bis etwa 3000 Basenpaare Länge untersucht, eine globale Untersuchung von Zellen auf Tausenden von möglichen Methylierungsanalysen ist nicht möglich. Allerdings kann auch dieses Verfahren keine sehr kleinen Fragmente aus geringen Probenmengen zuverlässig analysieren. Diese gehen trotz Diffusionsschutz durch die Matrix verloren.

Eine Übersicht über die weiteren bekannten Möglichkeiten, 5-Methylcytosine nachzuweisen, kann aus dem folgenden Übersichtsartikel entnommen werden: Rein, T., DePamphilis, M. L., Zorbas, H., Nucleic Acids Res. 1998, 26, 2255.

Die Bisulfit-Technik wird bisher bis auf wenige Ausnahmen (z. B. Zechnigk, M. et al., Eur. J. Hum. Gen. 1997, 5, 94-98) nur in der Forschung angewendet. Immer aber werden kurze, spezifische Stücke eines bekannten Gens nach einer Bisulfit-Behandlung amplifziert und entweder komplett sequenziert (Olek, A. und Walter, J., Nat. Genet. 1997, 17, 275-276) oder einzelne Cytosin-Positionen durch eine "Primer-Extension-Reaktion" (Gonzalgo, M. L. und Jones, P. A., Nucl. Acids Res. 1997, 25, 2529-2531, WO-Patent 9500669) oder einen Enzymschnitt (Xiong, Z. und Laird, P. W., Nucl. Acids. Res. 1997, 25, 2532-2534) nachgewiesen. Zudem ist auch der Nachweis durch Hybridisierung beschrieben worden (Olek et al., WO 99 28498).

Weitere Publikationen, die sich mit der Anwendung der Bisulfit-Technik zum Methylierungsnachweis bei einzelnen Genen befassen, sind: Xiong, Z. und Laird, P. W. (1997), Nucl. Acids Res. 25, 2532; Gonzalgo, M. L. und Jones, P. A. (1997), Nucl. Acids Res. 25, 2529; Grigg, S. und Clark, S. (1994), Bioassays 16, 431; Zeschnik, M. et al. (1997), Human Molecular Genetics 6, 387; Teil, R. et al. (1994), Nucl. Acids Res. 22, 695; Martin, V. et al. (1995), Gene 157, 261; WO 97 46705, WO 95 15373 und WO 45560.

Eine Übersicht über den Stand der Technik in der Oligomer Array Herstellung läßt sich aus einer im Januar 1999 erschienenen Sonderausgabe von Nature Genetics (Nature Genetics Supplement, Volume 21, January 1999), der dort zitierten Literatur und dem US-Patent 5994065 über Methoden zur Herstellung von festen Trägern für Zielmoleküle wie Oligonukleotide bei vermindertem nichtspezifischem Hintergrundsignal entnehmen.

Für die Abtastung eines immobilisierten DNA-Arrays sind vielfach fluoreszent markierte Sonden verwendet worden. Besonders geeignet für Fluoreszenzmarkierungen ist das einfache Anbringen von Cy3 und Cy5 Farbstoffen am 5'-OH der jeweiligen Sonde. Die Detektion der Fluoreszenz der hybridisierten Sonden erfolgt beispielsweise über ein Konfokalmikroskop. Die Farbstoffe Cy3 und Cy5 sind, neben vielen anderen, kommerziell erhältlich.

Matrix-assistierte Laser Desorptions/Ionisations-Massenspektrometrie (MALDI-TOF) ist eine sehr leistungsfähige Entwicklung für die Analyse von Biomolekülen (Karas, M. und Hillenkamp, F. (1988), Laser desorption ionization of proteins with molecular masses exeeding 10000 daltons. Anal. Chem. 60: 2299-2301). Ein Analyt wird in eine lichtabsorbierende Matrix eingebettet. Durch einen kurzen Laserpuls wird die Matrix verdampft und das Analytmolekül so unfragmentiert in die Gasphase befördert. Durch Stöße mit Matrixmolekülen wird die Ionisation des Analyten erreicht. Eine angelegte Spannung beschleunigt die Ionen in ein feldfreies Flugrohr. Auf Grund ihrer verschiedenen Massen werden Ionen unterschiedlich stark beschleunigt. Kleinere Ionen erreichen den Detektor früher als größere.

MALDI-TOF Spektroskopie eignet sich ausgezeichnet zur Analyse von Peptiden und Proteinen. Die Analyse von Nukleinsäuren ist etwas schwieriger (Gut, I. G. und Beck, S. (1995)), DNA and Matrix Assisted Laser Desorption Ionization Mass Spectrometry. Molecular Biology: Current Innovations and Future Trends 1: 147-157.) Für Nukleinsäuren ist die Empfindlichkeit etwa 100 mal schlechter als für Peptide und nimmt mit zunehmender Fragmentgröße überproportional ab. Für Nukleinsäuren, die ein vielfach negativ geladenes Rückgrat haben, ist der Ionisationsprozeß durch die Matrix wesentlich ineffizienter. In der MALDI-TOF Spektroskopie spielt die Wahl der Matrix eine eminent wichtige Rolle. Für die Desorption von Peptiden sind einige sehr leistungsfähige Matrices gefunden worden, die eine sehr feine Kristallisation ergeben. Für DNA gibt es zwar mittlererweile einige ansprechende Matrices, jedoch wurde dadurch der Empfindlichkeitsunterschied nicht verringert. Der Empfindlichkeitsunterschied kann verringert werden, indem die DNA chemisch so modifiziert wird, dass sie einem Peptid ähnlicher wird. Phosphorothioatnukleinsäuren, bei denen die gewöhnlichen Phosphate des Rückgrats durch Thiophosphate substituiert sind, lassen sich durch einfache Alkylierungschemie in eine ladungsneutrale DNA umwandeln (Gut, I. G. und Beck, S. (1995), A procedure for selective DNA alkylation and detection by mass spectrometry. Nucleic Acids Res. 23: 1367-1373). Die Kopplung eines "charge tags" an diese modifizierte DNA resultiert in der Steigerung der Empfindlichkeit um den gleichen Betrag, wie er für Peptide gefunden wird. Ein weiterer Vorteil von "charge tagging" ist die erhöhte Stabilität der Analyse gegen Verunreinigungen, die den Nachweis unmodifizierter Substrate stark erschweren.

Genomische DNA wird durch Standardmethoden aus DNA von Zell-, Gewebe- oder sonstigen Versuchsproben gewonnen. Diese Standardmethodik findet sich in Referenzen wie Fritsch und Maniatis eds., Molecular Cloning: A Laboratory Manual, 1989.

Harnstoff verbessert die Effizienz der Bisulfit-Behandlung vor der Sequenzierung von 5-Methylcytosin in genomischer DNA (Paulin R, Grigg GW, Davey MW, Piper AA.(1998), Nucleic Acids Res. 26: 5009-5010).

Aufgabe der vorliegende Erfindung ist es daher eine Verfahren zum Nachweis von Cytosin-Methylierungen in DNA zur Verfügung zu stellen, welches die Nachteile des Standes der Technik überwindet.

Die Aufgabe wird dadurch gelöst, dass ein Verfahren zum Nachweis von Cytosin-Methylierungen in DNA zur Verfügung gestellt wird, wobei man folgende Arbeitsschritte ausführt:
a) eine genomische DNA-Probe wird mit einer Lösung eines Bisulfits (= Hydrogensulfit, Disulfit) im Konzentrationsbereich zwischen 0,1 und 6 mol/l inkubiert, wobei ein denaturierendes Reagenz und/oder Lösemittel sowie mindestens ein Radikalfänger zugegen ist;
b) die behandelte DNA-Probe wird mit Wasser oder einer wässrigen Lösung verdünnt;
c) die DNA-Probe wird in einer Polymerasereaktion amplifiziert;
d) man detektiert, inwieweit sich die Sequenz durch die Behandlung nach Schritt a) gegenüber der genomischen DNA-Probe verändert hat und schließt auf den Methylierungsstatus zumindest eines Locus in der genomischen DNA-Probe.

Erfindungsgemäß bevorzugt ist es dabei, dass das denaturierende Reagenz und/oder Lösungsmittel aus der folgenden Liste von Verbindungen oder Verbindungsklassen ausgewählt ist:
Polyethylenglykoldialkylether, Dioxan und substituierte Derivate, Harnstoff oder Derivate, Acetonitril, primäre Alkohole, sekundäre Alkohole, tertiäre Alkohole, Diethylenglykoldialkylether, Triethylenglykoldialkylether, Tetraethylenglykol-dialkylether, Pentaethylenglykoldiakylether, Hexaethylenglykoldialkylether, DMSO, THF.

Bevorzugt ist dabei ferner, dass der Radikalfänger aus der folgenden Gruppe von Verbindungen ausgewählt ist:
Di-, Trihydroxybenzole, Grüntee Extrakt (green tea extract), Pycnogenol (pine bark extract), Ginkgo Biloba Extrakt (EGb 761), Flavonoid-Mischung verschiedener Frucht- und Gemüseextrakte (GNLD), Bio-Normalizer (Sun-O Corp), DPPH (1,1-Diphenyl-2-picrylhydrazyl), NDGA (Nordihydroguajaret-säure), Trolox (6-Hydroxy-2,5,7,8-tetramethylchroman-2-karbonsäure), 2,6-Di-tert-butylphenol, 4-Methyl-di-tert-butylphenol, 4-Methoxy-di-tert-butylphenol, 2,6-Di-tert-butyl-p-cresol, 3,4-Dihydroxybenzoesäure, Vitamin C, Vitamin E, Vitamin Q, Hydrochinon, Ubichinon, Lignane, Hydroxyterpene, Flavonoide, Curcumin, Tannine, Retinsäureverbindungen, Ge-132 Bisbetacarboxyethyl-germanium-sesquioxid, Superoxid-Dismutase (SOD), Superoxid-Katalase, Alpha-Naphthoflavon, Di(2-methyl-5-chlorophenyl)dithionat und Cu(II)-Derivate, Mebendazol, CS (chloroformlöslicher) Alkaloid-Extrakt,
   4-(3,5-Di-tert-butyl-4-hydroxyphenyl)-3-hydroxy-1,2-naphthochinon,
   4-(3,5-Di-tert-butyl-4-hydroxyphenyl)-3-methoxy-1,2-naphthochinon,
   4-(3,5-Di-tert-butyl-4-hydroxyphenyl)-1,2-naphthochinon,
   2-(3,5-Di-tert-butyl-4-hydroxyphenyl)-3-brom-1,4-naphthochinon,
   2-(3,5-Di-tert-butyl-4-hydroxyphenyl)-3-chlor-1,4-naphthochinon,
   2-(3,5-Di-tert-butyl-4-hydroxyphenyl)-3-methoxy-1,4-naphthochinon,
   2-(3,5-Di-tert-butyl-4-hydroxyphenyl)-3-hydroxy-1,4-naphthochinon,
   2-(3,5-Di-tert-butyl-4-hydroxyphenyl)-1,4-naphthochinon,
   4-(3,5-Di-tert-butyl-4-hydroxyphenyl)-3-hydroxy-5,5,8,8-tetramethyl-5,6,7,8-tetrahydro-1,2-anthrachinon,
   4-(3,5-Di-tert-butyl-4-hydroxyphenyl)-3-methoxy-5,5,8,8-tetramethyl-5,6,7,8-tetrahydro-1,2-anthrachinon,
   4-(3,5-Di-tert-butyl-4-hydroxyphenyl)-5,5,8,8-tetramethyl-5,6,7,8-tetrahydro-1,2-anthrachinon,
   3-Brom-4-(3,5-di-tert-butyl-4-hydroxyphenyl)-5,5,8,8-tetramethyl-5,6,7,8-tetrahydro-1,2-anthrachinon,
   2-(3,5-Di-tert-butyl-4-oxocyclohexa-2,5-dienyliden)-indan-1,3-dion,
   2-(3,5-Di-tert-butyl-4-oxocyclohexa-2,5-dienyliden)-3,4-epoxy-3-hydroxy-4-methoxy-3,4-dihydro-2H-naphthalin-1-on,
   2-(3,5-Di-tert-butyl-4-oxocyclohexa-2,5-dienyliden)-3,4-epoxy-3,4-dimethoxy-3,4-dihydro-2H-naphthalin-1-on,
   2-(3,5-Di-tert-butyl-4-hydroxyphenyl)-indan-1-on, 3,3-Bi-[2-(3,5-di-tert-butyl-4-hydroxyphenyl)-inden-1-on]-3-yl,
   2-(3,5-Di-tert-butyl-4-hydroxyphenyl)-3-brom-5,5,8,8-tetramethyl-5,6,7,8-tetrahydro-1,4-anthrachinon,
   2-(3,5-Di-tert-butyl-4-hydroxyphenyl)-3-chlor-5,5,8,8-tetramethyl-5,6,7,8-tetrahydro-1,4-anthrachinon,
   2-(3,5-Di-tert-butyl-4-hydroxyphenyl)-3-methoxy-5,5,8,8-tetramethyl-5,6,7,8-tetrahydro-1,4-anthrachinon,
   2-(3,5-Di-tert-butyl-4-hydroxyphenyl)-3-hydroxy-5,5,8,8-tetramethyl-5,6,7,8-tetrahydro-1,4-anthrachinon,
   2-(3,5-Di-tert-butyl-4-hydroxyphenyl)-5,5,8,8-tetramethyl-5,6,7,8-tetrahydro-1,4-anthrachinon,
   2-Brom-3-(3-brom-5-tert-butyl-4-hydroxyphenyl)-5,5,8,8-tetramethyl-5,6,7,8-tetrahydro-1,4-anthrachinon,
   2-Brom-3-(3,5-dibrom-4-hydroxyphenyl)-5,5,8,8-tetramethyl-5,6,7,8-tetrahydro-1,4-anthrachinon,
   2-Brom-3-(3-brom-5-tert-butyl-4-hydroxyphenyl)-3-hydroxy-5,5,8,8-tetramethyl-5,6,7,8-tetrahydro-1,4-anthrachinon,
   3-Brom-2-(3,5-di-tert-butyl-4-hydroxyphenyl)-1,4-anthrachinon,
   2-(3,5-Di-tert-butyl-4-hydroxyphenyl)-3-methoxy-1,4-anthrachinon,
   2-(3,5-Di-tert-butyl-4-hydroxyphenyl)-3-hydroxy-1,4-anthrachinon,
   5,5,8,8-Tetramethyl-5,6,7,8-tetrahydronaphthalin-1,3-diol,
   3-Methoxy-5,5,8,8-tetramethyl-5,6,7,8-tetrahydronaphthalin-1-ol,
   4-(3-Chlor-5,5,8,8-tetramethyl-1,4-dioxo-1,4,5,6,7,8-hexahydroanthracen-2-yl)-benzoesäure,
   Methyl-4-(3-chlor-5,5,8,8-tetramethyl-1,4-dioxo-1,4,5,6,7,8-hexahydroanthracen-2-yl)-benzoat,
   4-(3-Hydroxy-1,4-dioxo-1,4-dihydronaphthalin-2-yl)-benzoesäure,
   Methyl-(3-methoxy-1,4-dioxo-1,4-dihydronaphthalin-2-yl)-benzoesäure,
   4-(3-Hydroxy-5,5,8,8-tetramethyl-1,4-dioxo-1,4,5,6,7,8-hexahydroanthracen-2-yl)-benzoesäure,
   Methyl-4-(3-hydroxy-1,4-dioxo-1,4-dihydronaphthalin-2-yl-azo)-benzoat,
   4-(3-Hydroxy-5,5,8,8-tetramethyl-1,4-dioxo-1,4,5,6,7,8-hexahydroanthracen-2-yl-azo)-benzoesäure,
   3-(3,5-Di-tert-butyl-4-oxocyclohexa-2,5-dienyliden)-5,5,8,8-tetramethyl-5,6,7,8-tetrahydrocyclopenta[b]naphthalin-1,2-dion,
   3-(3,5-Di-tert-butyl-4-oxocyclohexa-2,5-dienyliden)-5,5,8,8-tetramethyl-5,6,7,8-tetrahydroanthracen-3H-1,2,4-trion,
   2-(3,5-Di-tert-butyl-4-hydroxyphenyl)-3-methoxy-5,8-dimethyl-1,4-naphthochinon,
   2-(3,5-Di-tert-butyl-4-hydroxyphenyl)-3-methoxy-6,7-dimethyl-1,4-naphthochinon,
   2-(3,5-Di-tert-butyl-4-hydroxyphenyl)-3-methoxy-5-methyl-1,4-naphthochinon,
   2-(3,5-Di-tert-butyl-4-hydroxyphenyl)-2-methoxy-5-methyl-1,4-naphthochinon,
   2-(3,5-Di-tert-butyl-4-hydroxyphenyl)-3-methoxy-6-methyl-1,4-naphthochinon,
   3-(3,5-Di-tert-butyl-4-hydroxyphenyl)-2-methoxy-6-methyl-1,4-naphthochinon,
   2-(3,5-Di-tert-butyl-4-hydroxyphenyl)-3-methoxy-5,6-dimethyl-1,4-naphthochinon,
   3-(3,5-Di-tert-butyl-4-hydroxyphenyl)-2-methoxy-5,6-dimethyl-1,4-naphthochinon,
   2-(3,5-Di-tert-butyl-4-hydroxyphenyl)-3-methoxy-5,7-dimethyl-1,4-naphthochinon,
   3-(3,5-Di-tert-butyl-4-hydroxyphenyl)-2-methoxy-5,7-dimethyl-1,4-naphthochinon,
   2-(3,5-Di-tert-butyl-4-hydroxyphenyl)-3-ethylthio-5-methyl-1,4-naphthochinon,
   2-(3,5-Di-tert-butyl-4-hydroxyphenyl)-3-ethylthio-6-methyl-1,4-naphthochinon,
   2-(3,5-Di-tert-butyl-4-hydroxyphenyl)-3-hydroxy-5,8-dimethyl-1,4-naphthochinon,
   2-(3,5-Di-tert-butyl-4-hydroxyphenyl)-3-hydroxy-6,7-dimethyl-1,4-naphthochinon,
   2-(3,5-Di-tert-butyl-4-hydroxyphenyl)-3-hydroxy-5-methyl-1,4-naphthochinon,
   3-(3,5-Di-tert-butyl-4-hydroxyphenyl)-2-hydroxy-5-methyl-1,4-naphthochinon,
   2-(3,5-Di-tert-butyl-4-hydroxyphenyl)-3-hydroxy-6-methyl-1,4-naphthochinon,
   3-(3,5-Di-tert-butyl-4-hydroxyphenyl)-2-hydroxy-6-methyl-1,4-naphthochinon,
   2-(3,5-Di-tert-butyl-4-hydroxyphenyl)-3-hydroxy-5,6-dimethyl-1,4-naphthochinon,
   2-(3-Brom-5-tert-butyl-4-hydroxyphenyl)-3-hydroxy-5,6-dimethyl-1,4-naphthochinon,
   3-(3,5-Di-tert-butyl-4-hydroxyphenyl)-2-hydroxy-5,6-dimethyl-1,4-naphthochinon,
   2-(3,5-Di-tert-butyl-4-hydroxyphenyl)-3-hydroxy-5,7-dimethyl-1,4-naphthochinon,
   3-(3,5-Di-tert-butyl-4-hydroxyphenyl)-2-hydroxy-5,7-dimethyl-1,4-naphthochinon.

Erfindungsgemäß bevorzugt ist dabei, dass man die genomische DNA-Probe vor der Behandlung thermisch denaturiert.

Besonders erfindungsgemäß bevorzugt ist es, dass man den Schritt c) in zwei Teilschritten wie folgt durchführt:
a) eine PCR Präamplifikation mit mindestens einem Primerpaar unterschiedlicher Sequenz, die an eine nach Anspruch 1 vorbehandelte DNA-Probe unspezifisch hybridisieren und daher im PCR Schritt mehr als ein Amplifikat ergeben;
b) eine PCR Amplifikation des in der Präamplifikation gebildeten Produkts mit Primern unterschiedlicher Sequenz, die jeweils zu einem Abschnitt der nach Anspruch 1 vorbehandelten DNA-Probe [(+)-Strang oder (-)-Strang] identisch oder revers komplementär sind und die zu amplifizierende DNA spezifisch hybridisieren.

Es ist erfindungsgemäß weiterhin bevorzugt, dass man die Amplifikation von mehreren DNA-Abschnitten in einem Reaktionsgefäß durchführt.

Bevorzugt ist es erfindungsgemäß außerdem, dass man für die Polymerasereaktion eine hitzebeständige DNA-Polymerase verwendet.

Besonders ist es erfindungsgemäß bevorzugt, dass man vor Schritt c) des erfindungsgemäßen Verfahrens eine Desulfonierung der DNA durchführt.

Es ist auch bevorzugt, dass man für die Detektion der vorbehandelten DNA die PCR-Produkte auf einen Oligonukleotid Array hybridisiert und man anschließend die folgenden Teilschritte ausführt:
a) die amplifizierte genomische DNA wird an mindestens ein Oligonukleotid unter Ausbildung einer Duplex hybridisiert, wobei besagte hybridisierte Oligonukleotide mit ihrem 3'-Ende unmittelbar oder im Abstand von bis zu 10 Basen an die Positionen angrenzen, die hinsichtlich ihrer Methylierung in der genomischen DNA-Probe zu untersuchen sind;
(b) man das Oligonukleotid mit bekannter Sequenz von n Nukleotiden mittels einer Polymerase mindestens um ein Nukleotid verlängert, wobei das Nukleotid eine nachweisbare Markierung trägt und die Verlängerung vom Methylierungsstatus des jeweiligen Cytosins in der genomischen DNA-Probe abhängt.

Es ist erfindungsgemäß bevorzugt, dass man für die Detektion der vorbehandelten DNA die PCR-Produkte auf einen Oligonukleotid Array hybridisiert und man anschließend die folgenden Teilschritte ausgeführt:
(a) man hybridisiert einen Satz von Oligonukleotiden an die amplifizierte genomische DNA unter Ausbildung einer Duplex, wobei dieser Satz von Oligonukleotiden aus zwei verschiedenen Spezies besteht und wobei die hybridisierten Oligonukleotide der ersten Spezies mit ihrem 3'-Ende unmittelbar oder im Abstand von bis zu 10 Basen an die Positionen angrenzen, die hinsichtlich ihrer Methylierung in der genomischen DNA-Probe zu untersuchen sind und wobei das zweite Oligonukleotid der zweiten Spezies an eine zweite Region des Zielmoleküls hybridisiert, so dass das 5'-Ende des Oligonukleotids der zweiten Spezies durch eine Lücke von der Größe eines Einzelnukleotides oder bis zu 10 Nukleotiden vom 3'-Ende des hybridisierten Oligonukleotids der ersten Spezies an der Stelle der besagten ausgewählten Position getrennt ist;
(b) man das Oligonukleotid der ersten Spezies mit bekannter Sequenz von n Nukleotiden mittels einer Polymerase um höchstens die Anzahl von Nukleotiden verlängert, die zwischen dem 3'-Ende des Oligonukleotids der 1. Spezies und dem 5'-Ende des Oligonukleotids der 2. Spezies liegen, wobei die Verlängerung vom Methylierungsstatus des jeweiligen Cytosins in der genomischen DNA-Probe abhängt;
(c) man inkubiert die Oligonukleotide in Gegenwart einer Ligase, wobei das angrenzende, durch die Polymerasereaktion verlängerte Oligonukleotid der ersten Spezies und das Oligonukleotid der zweiten Spezies verbunden werden und man dadurch ein Ligationsprodukt erhält, sofern im vorangehenden Schritt eine Verlängerung des Oligonukleotids der ersten Spezies derart erfolgte, dass nun das 3'-Ende mit vorhandener 3'-Hydroxyfunktion des verlängerten Oligonukleotids unmittelbar an das 5'-Ende des Oligonukleotids der zweiten Spezies angrenzt.

Dabei ist es erfindungsgemäß besonders bevorzugt, dass die verwendeten Oligonukleotide der ersten Spezies und/oder die verwendeten Oligonukleotide der zweiten Spezies entweder nur die Basen T, A und C oder aber die Basen T, A und G enthalten.

Es ist außerdem erfindungsgemäß bevorzugt, dass man für die Detektion der vorbehandelten DNA die PCR-Produkte auf einen Oligonukleotid Array hybridisiert und man anschließend die folgenden Teilschritte ausführt:
(a) man hybridisiert die amplifizierte genomische DNA an mindestens ein Oligonukleotid mit bekannter Sequenz von n Nukleotiden unter Ausbildung einer Duplex, wobei besagte hybridisierte Oligonukleotide mit ihrem 3'-Ende teilweise oder vollständig an die Positionen hybridisieren, die hinsichtlich ihrer Methylierung in der genomischen DNA-Probe zu untersuchen sind;
(b) man das Oligonukleotid, sofern es mit seinem 3'-Terminus zuvor ohne Basenfehlpaarungen an die zu untersuchenden Position hybridisierte, mittels einer Polymerase mindestens um ein Nukleotid verlängert, wobei mindestens ein Nukleotid eine nachweisbare Markierung trägt und die Verlängerung vom Methylierungsstatus des jeweiligen Cytosins in der genomischen DNA-Probe abhängt.

Es ist auch erfindungsgemäß bevorzugt, dass man die PCR-Produkte und/oder Verlängerungsprodukte und/oder Ligationsprodukte für die Detektion mit einer nachweisbaren Markierung versieht. Dabei ist es besonders bevorzugt, dass die Markierungen Fluoreszenzmarkierungen und/oder dass die Markierungen Radionuklide sind. Besonders bevorzugt ist es dabei, dass die Markierungen der Nukleotide ablösbare Massenmarkierungen sind, die in einem Massenspektrometer nachweisbar sind.

Insbesondere ist es auch bevorzugt, dass man die PCR-Produkte und/oder Verlängerungsprodukte und/oder Ligationsprodukte insgesamt im Massenspektrometer nachweist und somit durch ihre Masse eindeutig charakterisiert sind. Bevorzugt ist es erfindungsgemäß auch, dass man jeweils ein Fragment der PCR-Produkte und/oder Verlängerungsprodukte und/oder Ligationsprodukte im Massenspektrometer nachweist.

Das erfindungsgemäße Verfahren ist bevorzugt auch dadurch gekennzeichnet, dass man das Fragment des PCR-Produkts und/oder Verlängerungsprodukts und/oder Ligationsprodukts durch Verdau mit einer oder mehrerer Exo- oder Endonukleasen erzeugt.

Es ist weiterhin bevorzugt, dass man zur besseren Detektierbarkeit im Massenspektrometer die erzeugten Fragmente mit einer einzelnen positiven oder negativen Nettoladung versieht.

Ganz besonders bevorzugt ist es, dass man die PCR-Produkte und/oder Verlängerungsprodukte und/oder Ligationsprodukte mittels Matrix assistierter Laser Desorptions/Ionisations Massenspektrometrie (MALDI-TOF) oder mittels Elektrospray Massenspektrometrie (ESI) detektiert und visualisiert.

Das erfindungsgemäße Verfahren ist auch derart bevorzugt, in dem man die genomische DNA aus einer DNA-Probe erhält, wobei Quellen für DNA z. B. Zelllinien, Blut, Sputum, Stuhl, Urin, Gehirn-Rückenmarks-Flüssigkeit, in Paraffin einbettetes Gewebe, beispielsweise Gewebe von Augen, Darm, Niere, Hirn, Herz, Prostata, Lunge, Brust oder Leber, histologische Objektträger und alle möglichen Kombinationen hiervon umfassen.

Ein weiterer Gegenstand der vorliegenden Erfindung ist die Verwendung eines erfindungsgemäßen Verfahrens zur Diagnose und/oder Prognose nachteiliger Ereignisse für Patienten oder Individuen, wobei diese nachteiligen Ereignisse mindestens einer der folgenden Kategorien angehören: unerwünschte Arzneimittelwirkungen; Krebserkrankungen; CNS-Fehlfunktionen, Schäden oder Krankheit; Aggressionssymptome oder Verhaltensstörungen; klinische, psychologische und soziale Konsequenzen von Gehirnschädigungen; psychotische Störungen und Persönlichkeitsstörungen; Demenz und/oder assoziierte Syndrome; kardiovaskuläre Krankheit, Fehlfunktion und Schädigung; Fehlfunktion, Schädigung oder Krankheit des gastrointestinalen Traktes; Fehlfunktion, Schädigung oder Krankheit des Atmungssystems; Verletzung, Entzündung, Infektion, Immunität und/oder Rekonvaleszenz; Fehlfunktion, Schädigung oder Krankheit des Körpers als Abweichung im Entwicklungsprozess; Fehlfunktion, Schädigung oder Krankheit der Haut, der Muskeln, des Bindegewebes oder der Knochen; endokrine und metabolische Fehlfunktion, Schädigung oder Krankheit; Kopfschmerzen oder sexuelle Fehlfunktion.

Gegenstand der Erfindung ist auch die Verwendung eines erfindungsgemäßen Verfahrens zur Unterscheidung von Zelltypen oder Geweben oder zur Untersuchung der Zelldifferenzierung.

Ein weiterer Gegenstand der vorliegenden Erfindung ist schließlich ein Kit, bestehend aus einem Bisulfit enthaltenen Reagenz, denaturierenden Reagenzien oder Lösungsmitteln, sowie Radikalfängern und Primern zur Herstellung der Amplifikate, sowie eine Anleitung zur Durchführung eines Assays nach einem erfindungsgemäßen Verfahren.

Die vorliegende Erfindung stellt ein automatisierbares Verfahren zum Nachweis von Methylcytosin bereit, welches nur Pipettierschritte enthält. Dadurch wird die Effizienz bestehender Verfahren in Bezug auf die Einfachheit der Handhabung, die Qualität, die Kosten und vor allem den Durchsatz verbessert.

Beschrieben wird ein automatisierbares Verfahren zum Nachweis von Methylcytosin in genomischen DNA-Proben:

Die zu analysierende genomische DNA wird bevorzugt aus den üblichen Quellen für DNA erhalten, wie z. B. Zelllinien, Blut, Sputum, Stuhl, Urin, Gehirn-Rückenmarks-Flüssigkeit, in Paraffin eingebettetes Gewebe, beispielsweise Gewebe von Augen, Darm, Niere, Hirn, Herz, Prostata, Lunge, Brust oder Leber, histologische Objektträger und alle möglichen Kombinationen hiervon.

Im ersten Schritt des Verfahrens behandelt man die eingesetzte DNA bevorzugt mit Bisulfit, (= Disulfit, Hydrogensulfit) derart, dass alle nicht an der 5-Position der Base methylierten Cytosine so verändert werden, dass eine hinsichtlich dem Basenpaarungsverhalten unterschiedliche Base entsteht, während die in 5-Position methylierten Cytosine unverändert bleiben.

Die genomische DNA-Probe denaturiert man besonders bevorzugt vor der Behandlung thermisch.

Wird für die Reaktion Bisulfit im Konzentrationsbereich zwischen 0.1 und 6 mol/l verwendet, so findet an den nicht methylierten Cytosinbasen eine Addition statt. Für das erfindungsgemäße Verfahren müssen zudem ein denaturierendes Reagenz oder Lösungsmittel sowie ein Radikalfänger zugegen sein.

Dabei kommen als denaturierende Reagenzien oder Lösungsmittel bevorzugt die folgenden Verbindungen oder Verbindungsklassen in Frage:

Polyethylenglykoldialkylether, Dioxan und substituierte Derivate, Harnstoff oder Derivate, Acetonitril, primäre Alkohole, sekundäre Alkohole, tertiäre Alkohole, Diethylenglykoldialkylether, Triethylenglykoldialkylether, Tetraethylenglykol-dialkylether, Pentaethylenglykoldiakylether, Hexaethylenglykoldialkylether, DMSO oder THF.

Als Radikalfänger ist die Gruppe der in Liste 1 aufgelisteten Verbindungen oder deren Derivate vorzugsweise geeignet. Die anschließende alkalische Hydrolyse führt dann zur Umwandlung von nicht methylierten Cytosin-Nukleobasen in Uracil.

Im zweiten Verfahrensschritt verdünnt man die behandelte DNA-Probe mit Wasser oder einer wässrigen Lösung. Bevorzugt wird anschließend eine Desulfonierung der DNA (10-30 min, 90-100 °C) bei alkalischem pH-Wert durchgeführt.

Im dritten Schritt des Verfahrens amplifiziert man die DNA-Probe in einer Polymerasekettenreaktion, bevorzugt mit einer hitzebeständigen DNA-Polymerase. Die Amplifikation von mehreren DNA-Abschnitten wird vorzugsweise in einem Reaktionsgefäß gemacht.

Den Verfahrensschritt führt man vorzugsweise in zwei Teilschritten durch. Man beginnt mit einer PCR Präamplifikation mit mindestens einem Primerpaar unterschiedlicher Sequenz, welche die vorbehandelte DNA Probe unspezifisch hybridisieren und daher im PCR Schritt mehr als ein Amplifkat ergeben. Danach führt man eine PCR Amplifikation des in der Präamplifikation gebildeten Produkts mit Primern unterschiedlicher Sequenz durch, die jeweils zu einem Abschnitt der vorbehandelten DNA-Probe [(+)-Strang oder (-)-Strang] identisch oder revers komplementär sind und die zu amplifizierende DNA spezifisch hybridisieren.

Dabei ist es klar, dass derartige Präamplifikationen häufig nicht als PCR Reaktionen, sondern als Primerextensionsreaktionen ausgeführt werden, die keine hitzebeständige Polymerase erfordern.

Im letzten Verfahrensschritt detektiert man, inwieweit sich die Sequenz durch die Behandlung mit einem Bisulfit enthaltenen Reagenz gegenüber der genomischen DNA-Probe verändert hat und schließt auf den Methylierungsstatus zumindest eines Locus in der genomischen DNA-Probe.

Für die Detektion werden die PCR-Produkte besonders bevorzugt auf einen Oligonukleotid Array hybridisiert.

In einer bevorzugten Variante des Verfahrens führt man nach der Hybridisierung auf einen Oligonukleotid Array die folgenden Teilschritte durch:
a) die amplifizierte genomische DNA wird an mindestens ein Oligonukleotid unter Ausbildung einer Duplex hybridisiert, wobei besagte hybridisierte Oligonukleotide mit ihrem 3'-Ende unmittelbar oder im Abstand von bis zu 10 Basen an die Positionen angrenzen, die hinsichtlich ihrer Methylierung in der genomischen DNA-Probe zu untersuchen sind;
(b) das Oligonukleotid mit bekannter Sequenz von n Nukleotiden wird mittels einer Polymerase mindestens um ein Nukleotid verlängert, wobei das Nukleotid eine nachweisbare Markierung trägt und die Verlängerung vom Methylierungsstatus des jeweiligen Cytosins in der genomischen DNA-Probe abhängt.

In einer weiteren bevorzugten Variante des Verfahrens führt man nach der Hybridisierung auf einen Oligonukleotid Array die folgenden Teilschritte durch:
(a) man hybridisiert einen Satz von Oligonukleotiden an die amplifizierte genomische DNA unter Ausbildung einer Duplex, wobei dieser Satz von Oligonukleotiden aus zwei verschiedenen Spezies besteht und wobei die hybridisierten Oligonukleotide der ersten Spezies mit ihrem 3'-Ende unmittelbar oder im Abstand von bis zu 10 Basen an die Positionen angrenzen, die hinsichtlich ihrer Methylierung in der genomischen DNA-Probe zu untersuchen sind und wobei das zweite Oligonukleotid der zweiten Spezies an eine zweite Region des Zielmoleküls hybridisiert, so dass das 5'-Ende des Oligonukleotids der zweiten Spezies durch eine Lücke von der Größe eines Einzelnukleotides oder bis zu 10 Nukleotiden vom 3'-Ende des hybridisierten Oligonukleotids der ersten Spezies an der Stelle der besagten ausgewählten Position getrennt ist;
(b) das Oligonukleotid der ersten Spezies mit bekannter Sequenz von n Nukleotiden wird mittels einer Polymerase um höchstens die Anzahl von Nukleotiden verlängert, die zwischen dem 3'-Ende des Oligonukleotids der 1. Spezies und dem 5'-Ende des Oligonukleotids der 2. Spezies liegen, wobei die Verlängerung vom Methylierungsstatus des jeweiligen Cytosins in der genomischen DNA-Probe abhängt;
(c) man inkubiert die Oligonukleotide in Gegenwart einer Ligase, wobei das angrenzende, durch die Polymerasereaktion verlängerte Oligonukleotid der ersten Spezies und das Oligonukleotid der zweiten Spezies verbunden werden und man dadurch ein Ligationsprodukt erhält, sofern im vorangehenden Schritt eine Verlängerung des Oligonukleotids der ersten Spezies derart erfolgte, dass nun das 3'-Ende mit vorhandener 3'-Hydroxyfunktion des verlängerten Oligonukleotids unmittelbar an das 5'-Ende des Oligonukleotids der zweiten Spezies angrenzt, welches bevorzugt phosphoryliert vorliegt.

Die verwendeten Oligonukleotide der ersten Spezies und/oder die verwendeten Oligonukleotide der zweiten Spezies enthalten besonders bevorzugt entweder nur die Basen T, A und C oder aber die Basen T, A und G.

In einer wiederum bevorzugten Variante des Verfahrens führt man nach der Hybridisierung auf einen Oligonukleotid Array die folgenden Teilschritte durch:
(a) man hybridisiert die amplifizierte genomische DNA an mindestens ein Oligonukleotid mit bekannter Sequenz von n Nukleotiden unter Ausbildung einer Duplex, wobei besagte hybridisierte Oligonukleotide mit ihrem 3'-Ende teilweise oder vollständig an die Positionen hybridisieren, die hinsichtlich ihrer Methylierung in der genomischen DNA-Probe zu untersuchen sind;
(b) das Oligonukleotid wird, sofern es mit seinem 3'Terminus zuvor ohne Basenfehlpaarungen an die zu untersuchenden Position hybridisierte, mittels einer Polymerase mindestens um ein Nukleotid verlängert, wobei mindestens ein Nukleotid eine nachweisbare Markierung trägt und die Verlängerung vom Methylierungsstatus des jeweiligen Cytosins in der genomischen DNA-Probe abhängt.

Die PCR-Produkte und/oder Verlängerungsprodukte und/oder Ligationsprodukte sind für die Detektion besonders bevorzugt mit einer nachweisbaren Markierung versehen.

Vorzugsweise sind die Markierungen der PCR-Produkte und/oder Verlängerungsprodukte und/oder Ligationsprodukte Fluoreszenzmarkierungen, Radionuklide oder ablösbare Massenmarkierungen, die in einem Massenspektrometer nachgewiesen werden.

Die PCR-Produkte und/oder Verlängerungsprodukte und/oder Ligationsprodukte können vorzugsweise insgesamt im Massenspektrometer nachgewiesen werden und sind somit durch ihre Masse eindeutig charakterisiert.

Besonders bevorzugt weist man jeweils ein Fragment der PCR-Produkte und/oder Verlängerungsprodukte und/oder Ligationsprodukte im Massenspektrometer nach.

Das Fragment des PCR-Produkts und/oder Verlängerungsprodukts und/oder Ligationsprodukts erzeugt man bevorzugt durch Verdau mit einer oder mehrerer Exo- oder Endonukleasen.

Zur besseren Detektierbarkeit im Massenspektrometer weisen die erzeugten Fragmente besonders bevorzugt eine einzelne positive oder negative Nettoladung auf.

Die PCR-Produkte und/oder Verlängerungsprodukte und/oder Ligationsprodukte detektiert und visualisiert man vorzugsweise mittels Matrix assistierter Laser Desorptions/Ionisations Massenspektrometrie (MALDI-TOF) oder mittels Elektrospray Massenspektrometrie (ESI).

Das vorliegende Verfahren wird bevorzugt verwendet zur Diagnose und/oder Prognose von nachteiligen Ereignisse für Patienten oder Individuen, wobei diese nachteiligen Ereignisse mindestens einer der folgenden Kategorien angehören: unerwünschte Arzneimittelwirkungen; Krebserkrankungen; CNS-Fehlfunktionen, Schäden oder Krankheit; Aggressionssymptome oder Verhaltensstörungen; klinische, psychologische und soziale Konsequenzen von Gehirnschädigungen; psychotische Störungen und Persönlichkeitsstörungen; Demenz und/oder assoziierte Syndrome; kardiovaskuläre Krankheit, Fehlfunktion und Schädigung; Fehlfunktion, Schädigung oder Krankheit des gastrointestinalen Traktes; Fehlfunktion, Schädigung oder Krankheit des Atmungssystems; Verletzung, Entzündung, Infektion, Immunität und/oder Rekonvaleszenz; Fehlfunktion, Schädigung oder Krankheit des Körpers als Abweichung im Entwicklungsprozess; Fehlfunktion, Schädigung oder Krankheit der Haut, der Muskeln, des Bindegewebes oder der Knochen; endokrine und metabolische Fehlfunktion, Schädigung oder Krankheit; Kopfschmerzen oder sexuelle Fehlfunktion.

Das neue Verfahren dient ferner besonders bevorzugt zur Unterscheidung von Zelltypen, Geweben oder zur Untersuchung der Zelldifferenzierung.

Gegenstand der vorliegenden Erfindung ist ferner ein Kit, das ein Bisulfit enthaltenes Reagenz, denaturierende Reagenzien oder Lösungsmittel, sowie Radikalfänger gemäss Liste 1, Primer zur Herstellung der Amplifikate und eine Anleitung zur Durchführung eines Assays enthält.

### Liste 1:

Di-, Trihydroxybenzole, Grüntee Extrakt (green tea extract), Pycnogenol (pine bark extract), Ginkgo Biloba Extrakt (EGb 761), Flavonoid-Mischung verschiedener Frucht- und Gemüseextrakte (GNLD), Bio-Normalizer (Sun-O Corp),
DPPH (1,1-Diphenyl-2-picrylhydrazyl), NDGA (Nordihydroguajaret-säure),
Trolox (6-Hydroxy-2,5,7,8-tetramethylchroman-2-karbonsäure),
2,6-Di-tert-butylphenol, 4-Methyl-di-tert-butylphenol, 4-Methoxy-di-tert-butylphenol, 2,6-Di-tert-butyl-p-cresol, 3,4-Dihydroxybenzoesäure, Vitamin C, Vitamin E, Vitamin Q, Hydrochinon, Ubichinon, Lignane, Hydroxyterpene, Flavonoide, Curcumin, Tannine, Retinsäureverbindungen, Ge-132 Bisbetacarboxyethyl-germanium-sesquioxid, Superoxid-Dismutase (SOD), Superoxid-Katalase, Alpha-Naphthoflavon, Di(2-methyl-5-chlorophenyl)dithionat und Cu(II)-Derivate, Mebendazol, CS (chloroformlöslicher) Alkaloid-Extrakt,
4-(3,5-Di-tert-butyl-4-hydroxyphenyl)-3-hydroxy-1,2-naphthochinon,
4-(3,5-Di-tert-butyl-4-hydroxyphenyl)-3-methoxy-1,2-naphthochinon,
4-(3,5-Di-tert-butyl-4-hydroxyphenyl)-1,2-naphthochinon,
2-(3,5-Di-tert-butyl-4-hydroxyphenyl)-3-brom-1,4-naphthochinon,
2-(3,5-Di-tert-butyl-4-hydroxyphenyl)-3-chlor-1,4-naphthochinon,
2-(3,5-Di-tert-butyl-4-hydroxyphenyl)-3-methoxy-1,4-naphthochinon,
2-(3,5-Di-tert-butyl-4-hydroxyphenyl)-3-hydroxy-1,4-naphthochinon,
2-(3,5-Di-tert-butyl-4-hydroxyphenyl)-1,4-naphthochinon,
4-(3,5-Di-tert-butyl-4-hydroxyphenyl)-3-hydroxy-5,5,8,8-tetramethyl-5,6,7,8-tetrahydro-1,2-anthrachinon,
4-(3,5-Di-tert-butyl-4-hydroxyphenyl)-3-methoxy-5,5,8,8-tetramethyl-5,6,7,8-tetrahydro-1,2-anthrachinon,
4-(3,5-Di-tert-butyl-4-hydroxyphenyl)-5,5,8,8-tetramethyl-5,6,7,8-tetrahydro-1,2-anthrachinon,
3-Brom-4-(3,5-di-tert-butyl-4-hydroxyphenyl)-5,5,8,8-tetramethyl-5,6,7,8-tetrahydro-1,2-anthrachinon,
2-(3,5-Di-tert-butyl-4-oxocyclohexa-2,5-dienyliden)-indan-1,3-dion,
2-(3,5-Di-tert-butyl-4-oxocyclohexa-2,5-dienyliden)-3,4-epoxy-3-hydroxy-4-methoxy-3,4-dihydro-2H-naphthalin-1-on,
2-(3,5-Di-tert-butyl-4-oxocyclohexa-2,5-dienyliden)-3,4-epoxy-3,4-dimethoxy-3,4-dihydro-2H-naphthalin-1-on,
2-(3,5-Di-tert-butyl-4-hydroxyphenyl)-indan-1-on, 3,3-Bi-[2-(3,5-di-tert-butyl-4-hydroxyphenyl)-inden-1-on]-3-yl,
2-(3,5-Di-tert-butyl-4-hydroxyphenyl)-3-brom-5,5,8,8-tetramethyl-5,6,7,8-tetrahydro-1,4-anthrachinon,
2-(3,5-Di-tert-butyl-4-hydroxyphenyl)-3-chlor-5,5,8,8-tetramethyl-5,6,7,8-tetrahydro-1,4-anthrachinon,
2-(3,5-Di-tert-butyl-4-hydroxyphenyl)-3-methoxy-5,5,8,8-tetramethyl-5,6,7,8-tetrahydro-1,4-anthrachinon,
2-(3,5-Di-tert-butyl-4-hydroxyphenyl)-3-hydroxy-5,5,8,8-tetramethyl-5,6,7,8-tetrahydro-1,4-anthrachinon,
2-(3,5-Di-tert-butyl-4-hydroxyphenyl)-5,5,8,8-tetramethyl-5,6,7,8-tetrahydro-1,4-anthrachinon,
2-Brom-3-(3-brom-5-tert-butyl-4-hydroxyphenyl)-5,5,8,8-tetramethyl-5,6,7,8-tetrahydro-1,4-anthrachinon,
2-Brom-3-(3,5-dibrom-4-hydroxyphenyl)-5,5,8,8-tetramethyl-5,6,7,8-tetrahydro-1,4-anthrachinon,
2-Brom-3-(3-brom-5-tert-butyl-4-hydroxyphenyl)-3-hydroxy-5,5,8,8-tetramethyl-5,6,7,8-tetrahydro-1,4-anthrachinon,
3-Brom-2-(3,5-di-tert-butyl-4-hydroxyphenyl)-1,4-anthrachinon,
2-(3,5-Di-tert-butyl-4-hydroxyphenyl)-3-methoxy-1,4-anthrachinon,
2-(3,5-Di-tert-butyl-4-hydroxyphenyl)-3-hydroxy-1,4-anthrachinon,
5,5,8,8-Tetramethyl-5,6,7,8-tetrahydronaphthalin-1,3-diol,
3-Methoxy-5,5,8,8-tetramethyl-5,6,7,8-tetrahydronaphthalin-1-ol,
4-(3-Chlor-5,5,8,8-tetramethyl-1,4-dioxo-1,4,5,6,7,8-hexahydroanthracen-2-yl)-benzoesäure,
Methyl-4-(3-chlor-5,5,8,8-tetramethyl-1,4-dioxo-1,4,5,6,7,8-hexahydroanthracen-2-yl)-benzoat,
4-(3-Hydroxy-1,4-dioxo-1,4-dihydronaphthalin-2-yl)-benzoesäure,
Methyl-(3-methoxy-1,4-dioxo-1,4-dihydronaphthalin-2-yl)-benzoesäure,
4-(3-Hydroxy-5,5,8,8-tetramethyl-1,4-dioxo-1,4,5,6,7,8-hexahydroanthracen-2-yl)-benzoesäure,
Methyl-4-(3-hydroxy-1,4-dioxo-1,4-dihydronaphthalin-2-yl-azo)-benzoat,
4-(3-Hydroxy-5,5,8,8-tetramethyl-1,4-dioxo-1,4,5,6,7,8-hexahydroanthracen-2-yl-azo)-benzoesäure,
3-(3,5-Di-tert-butyl-4-oxocyclohexa-2,5-dienyliden)-5,5,8,8-tetramethyl-5,6,7,8-tetrahydrocyclopenta[b]naphthalin-1,2-dion,
3-(3,5-Di-tert-butyl-4-oxocyclohexa-2,5-dienyliden)-5,5,8,8-tetramethyl-5,6,7,8-tetrahydroanthracen-3H-1,2,4-trion,
2-(3,5-Di-tert-butyl-4-hydroxyphenyl)-3-methoxy-5,8-dimethyl-1,4-naphthochinon,
2-(3,5-Di-tert-butyl-4-hydroxyphenyl)-3-methoxy-6,7-dimethyl-1,4-naphthochinon,
2-(3,5-Di-tert-butyl-4-hydroxyphenyl)-3-methoxy-5-methyl-1,4-naphthochinon,
2-(3,5-Di-tert-butyl-4-hydroxyphenyl)-2-methoxy-5-methyl-1,4-naphthochinon,
2-(3,5-Di-tert-butyl-4-hydroxyphenyl)-3-methoxy-6-methyl-1,4-naphthochinon,
3-(3,5-Di-tert-butyl-4-hydroxyphenyl)-2-methoxy-6-methyl-1,4-naphthochinon,
2-(3,5-Di-tert-butyl-4-hydroxyphenyl)-3-methoxy-5,6-dimethyl-1,4-naphthochinon,
3-(3,5-Di-tert-butyl-4-hydroxyphenyl)-2-methoxy-5,6-dimethyl-1,4-naphthochinon,
2-(3,5-Di-tert-butyl-4-hydroxyphenyl)-3-methoxy-5,7-dimethyl-1,4-naphthochinon,
3-(3,5-Di-tert-butyl-4-hydroxyphenyl)-2-methoxy-5,7-dimethyl-1,4-naphthochinon,
2-(3,5-Di-tert-butyl-4-hydroxyphenyl)-3-ethylthio-5-methyl-1,4-naphthochinon,
2-(3,5-Di-tert-butyl-4-hydroxyphenyl)-3-ethylthio-6-methyl-1,4-naphthochinon,
2-(3,5-Di-tert-butyl-4-hydroxyphenyl)-3-hydroxy-5,8-dimethyl-1,4-naphthochinon,
2-(3,5-Di-tert-butyl-4-hydroxyphenyl)-3-hydroxy-6,7-dimethyl-1,4-naphthochinon,
2-(3,5-Di-tert-butyl-4-hydroxyphenyl)-3-hydroxy-5-methyl-1,4-naphthochinon,
3-(3,5-Di-tert-butyl-4-hydroxyphenyl)-2-hydroxy-5-methyl-1,4-naphthochinon,
2-(3,5-Di-tert-butyl-4-hydroxyphenyl)-3-hydroxy-6-methyl-1,4-naphthochinon,
3-(3,5-Di-tert-butyl-4-hydroxyphenyl)-2-hydroxy-6-methyl-1,4-naphthochinon,
2-(3,5-Di-tert-butyl-4-hydroxyphenyl)-3-hydroxy-5,6-dimethyl-1,4-naphthochinon,
2-(3-Brom-5-tert-butyl-4-hydroxyphenyl)-3-hydroxy-5,6-dimethyl-1,4-naphthochinon,
3-(3,5-Di-tert-butyl-4-hydroxyphenyl)-2-hydroxy-5,6-dimethyl-1,4-naphthochinon,
2-(3,5-Di-tert-butyl-4-hydroxyphenyl)-3-hydroxy-5,7-dimethyl-1,4-naphthochinon,
3-(3,5-Di-tert-butyl-4-hydroxyphenyl)-2-hydroxy-5,7-dimethyl-1,4-naphthochinon.

Das folgende Beispiel erläutert die Erfindung.

### Beispiel:

### Automatisierte Durchführung der Bisulfitreaktion.

Im vorliegenden Beispiel wird die Anwendung des Verfahrens zum Nachweis des Methylierungsstatus von Cytosinen im Faktor VIII-Gen einer genomischen DNA-Probe, die mit einer Restriktionsendonuclease nach Angabe des Herstellers behandelt wurde, beschrieben. Das Verfahren beruht auf dem Einsatz eines automatischen Pipettiersystems (MWG RoboSeq 4204) mit vier separat vertikal beweglichen Adaptern für austauschbare Pipettierspitzen, die Kreuzkontaminationen ausschließen. Das Pipettiersystem ermöglicht das Pipettieren von 100µl mit einem Fehler von weniger als ±2µl. Die Arbeitsplatte des automatischen Pipettiersystems ist mit sechs Gestellen für Pipettierspitzen und acht Pipettierpositionen, von denen zwei gekühlt werden können, einem kühlbaren Reagenziengestell, einem Stapelsystem für 10 Mikrotiterplatten, einer Pipettierspitzenwaschstation und einer Vorrichtung zur Trennung der Pipettierspitzen vom Adaptor ausgerüstet. Das automatische Pipettiersystem ist über eine serielle Schnittstelle mit einem Computer verbunden und wird über ein Softwareprogramm, das die freie Programmierung aller für zur Anwendung des Verfahrens notwendigen Pipettierschritte erlaubt, gesteuert.

Im ersten Verfahrensschritt wird von Hand ein Aliquot der DNA-Probe in eine von 96 frei wählbaren Positionen einer Mikrotiterplatte pipettiert. Die Mikrotiterplatte wird anschließend unter Verwendung eines Eppendorf Mastercyclers zur Denaturierung der vorbehandelten DNA-Probe auf 96°C erwärmt. Die Mikrotiterplatte wird dann in das automatische Pipettiersystem überführt. In alle Positionen, die DNA enthalten, werden programmgesteuert nacheinander aus dem Reagenziengestell Aliquots eines denaturierenden Agens (Dioxan), einer 3,3 molaren Natriumhydrogensulfitlösung, und einer Lösung eines Radikalfängers in dem verwendeten denaturierenden Agens hinzu pipettiert. Anschließend wird die Mikrotiterplatte im Eppendorf Mastercycler inkubiert, dass in der DNA-Probe unter Einwirkung des Natriumhydrogensulfits alle unmethylierten Cytosinreste in ein Bisulfitaddukt umgewandelt werden.

Nach der Bisulfitbehandlung wird die Mikrotiterplatte aus dem Thermocycler in das automatische Pipetiersystem überführt. Es wird eine zweite Mikrotiterplatte desselben Typs vorgelegt. In alle Kammern, deren äquivalente Position auf der ersten Mikrotiterplatte eine bisulfitbehandelte DNA-Probe enthält, wird zuerst ein basischer Tris-HCl Puffer (pH 9.5) und anschließend wird ein Aliquot der bisulfitbehandelten DNA in die entsprechende Position der zweiten Mikrotiterplatte übertragen. In der basischen Lösung werden die Bisulfitaddukte der nichtmethylierten Cytosinreste zu Uracilresten umgewandelt.

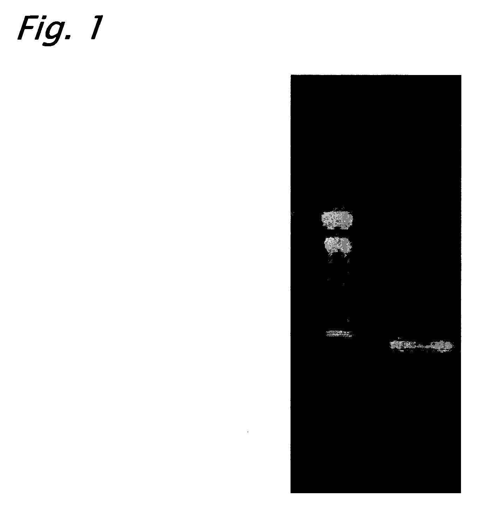

Die gezielte Amplifikation eines Stranges (im vorliegenden Beispiel der sense-Strang) der bisulfitbehandelten DNA erfolgt durch eine Polymerasekettenreaktion (PCR). Es wird ein Primerpaar des Typs 1 (AGG GAG TTT TTT TTA GGG AAT AGA GGG A (SEQ-ID:1) und TAA TCC CAA AAC CTC TCC ACT ACA ACA A (SEQ-ID:2)) verwendet, das die spezifische Amplifikation eines erfolgreich bisulfitbehandelten DNA-Strangs, nicht jedoch eines DNA-Strangs, dessen nichtmetylierte Cytosinreste nicht oder unvollständig in Uracilreste umgewandelt wurden, erlaubt. Für die PCR-Reaktion wird im automatischen Pipettiersystem eine dritte Mikrotiterplatte desselben Typs vorgelegt. In alle Kammern, deren äquivalente Positionen auf der ersten Mikrotiterplatte eine bisulfitbehandelte DNA-Probe enthält, wird zuerst ein Aliquot einer Stammlösung, die einen PCR-Puffer, eine DNA-Polymerase und Primer des Typs 1 enthält, automatisch pipettiert. Danach wird automatisch aus jeder Position der zweiten Mikrotiterplatte ein Aliquot der verdünnten bisulfitbehandelten DNA in die entsprechende Position der dritten Mikrotiterplatte übertragen, bevor diese zur Durchführung der PCR-Reaktion in den Cycler überführt wird. Das PCR-Produkt wird durch Agarosegelelektrophorese und anschließende Anfärbung mit Ethidiumbromid identifiziert (Fig. 1). Figur 1 zeigt das Gelbild eines PCR-amplifizierten bisulfitbehandelten DNA-Stranges (links: Molekulargewichtsmarker, rechts: PCR-Produkt)

## Patentansprüche

1. Verfahren zum Nachweis von Cytosin-Methylierungen in DNA, **dadurch gekennzeichnet, dass** man folgende Arbeitsschritte ausführt:
a) eine genomische DNA-Probe wird mit einer Lösung eines Bisulfits (= Hydrogensulfit, Disulfit) im Konzentrationsbereich zwischen 0,1 und 6 mol/l inkubiert, wobei ein denaturierendes Reagenz sowie mindestens ein Radikalfänger zugegen ist;
b) die behandelte DNA-Probe wird mit Wasser oder einer wässrigen Lösung verdünnt;
c) die DNA-Probe wird in einer Polymerasereaktion amplifiziert;
d) wobei vor Schritt c) eine Desulfonierung der DNA erfolgt;
e) man detektiert, inwieweit sich die Sequenz durch die Behandlung nach Schritt a) gegenüber der genomischen DNA-Probe verändert hat und schließt auf den Methylierungsstatus zumindest eines Locus in der genomischen DNA-Probe;
f) wobei die Schritte a - c unter Einsatz eines automatischen Pipetiersystems erfolgen.

2. Verfahren nach Anspruch 1, **dadurch gekennzeichnet, dass** das denaturierende Reagenz Harnstoff oder eines seiner Derivate ist.

3. Verfahren nach Anspruch 1 oder 2, **dadurch gekennzeichnet, dass** der Radikalfänger aus der folgenden Gruppe von Verbindungen ausgewählt ist:
Di-, Trihydroxybenzole, Grüntee Extrakt (green tea extract), Pycnogenol (pine bark extract), Ginkgo Biloba Extrakt (EGb 761), Flavonoid-Mischung verschiedener Frucht- und Gemüseextrakte (GNLD), Bio-Normalizer (Sun-O Corp), DPPH (1,1-Diphenyl-2-picrylhydrazyl), NDGA (Nordihydroguajaret-säure), Trolox (6-Hydroxy-2,5,7,8-tetramethylchroman-2-karbonsäure), 2,6-Di-tert-butylphenol, 4-Methyl-di-tert-butylphenol, 4-Methoxy-di-tert-butylphenol, 2,6-Di-tert-butyl-p-cresol, 3,4-Dihydroxybenzoesäure, Vitamin C, Vitamin E, Vitamin Q, Hydrochinon, Ubichinon, Lignane, Hydroxyterpene, Flavonoide, Curcumin, Tannine, Retinsäureverbindungen, Ge-132 Bisbetacarboxyethyl-germanium-sesquioxid, Superoxid-Dismutase (SOD), Superoxid-Katalase, Alpha-Naphthoflavon, Di(2-methyl-5-chlorophenyl)dithionat und Cu(II)-Derivate, Mebendazole, CS (chloroformlöslicher) Alkaloid-Extrakt,
4-(3,5-Di-tert-butyl-4-hydroxyphenyl)-3-hydroxy-1,2-naphthochinon,
4-(3,5-Di-tert-butyl-4-hydroxyphenyl)-3-methoxy-1,2-naphthochinon,
4-(3,5-Di-tert-butyl-4-hydroxyphenyl)-1,2-naphthochinon,
2-(3,5-Di-tert-butyl-4-hydroxyphenyl)-3-brom-1,4-naphthochinon,
2-(3,5-Di-tert-butyl-4-hydroxyphenyl)-3-chlor-1,4-naphthochinon,
2-(3,5-Di-tert-butyl-4-hydroxyphenyl)-3-methoxy-1,4-naphthochinon,
2-(3,5-Di-tert-butyl-4-hydroxyphenyl)-3-hydroxy-1,4-naphthochinon,
2-(3,5-Di-tert-butyl-4-hydroxyphenyl)-1,4-naphthochinon,
4-(3,5-Di-tert-butyl-4-hydroxyphenyl)-3-hydroxy-5,5,8,8-tetramethyl-5,6,7,8-tetrahydro-1,2-anthrachinon,
4-(3,5-Di-tert-butyl-4-hydroxyphenyl)-3-methoxy-5,5,8,8-tetramethyl-5,6,7,8-tetrahydro-1,2-anthrachinon,
4-(3,5-Di-tert-butyl-4-hydroxyphenyl)-5,5,8,8-tetramethyl-5,6,7,8-tetrahydro-1,2-anthrachinon,
3-Brom-4-(3,5-di-tert-butyl-4-hydroxyphenyl)-5,5,8,8-tetramethyl-5,6,7,8-tetrahydro-1,2-anthrachinon,
2-(3,5-Di-tert-butyl-4-oxocyclohexa-2,5-dienyliden)-indan-1,3-dion,
2-(3,5-Di-tert-butyl-4-oxocyclohexa-2,5-dienyliden)-3,4-epoxy-3-hydroxy-4-methoxy-3,4-dihydro-2H-naphthalin-1-on,
2-(3,5-Di-tert-butyl-4-oxocyclohexa-2,5-dienyliden)-3,4-epoxy-3,4-dimethoxy-3,4-dihydro-2H-naphthalin-1-on,
2-(3,5-Di-tert-butyl-4-hydroxyphenyl)-indan-1-on,
3,3-Bi-[2-(3,5-di-tert-butyl-4-hydroxyphenyl)-inden-1-on]-3-yl,
2-(3,5-Di-tert-butyl-4-hydroxyphenyl)-3-brom-5,5,8,8-tetramethyl-5,6,7,8-tetrahydro-1,4-anthrachinon,
2-(3,5-Di-tert-butyl-4-hydroxyphenyl)-3-chlor-5,5,8,8-tetramethyl-5,6,7,8-tetrahydro-1,4-anthrachinon,
2-(3,5-Di-tert-butyl-4-hydroxyphenyl)-3-methoxy-5,5,8,8-tetramethyl-5,6,7,8-tetrahydro-1,4-anthrachinon,
2-(3,5-Di-tert-butyl-4-hydroxyphenyl)-3-hydroxy-5,5,8,8-tetramethyl-5,6,7,8-tetrahydro-1,4-anthrachinon,
2-(3,5-Di-tert-butyl-4-hydroxyphenyl)-5,5,8,8-tetramethyl-5,6,7,8-tetrahydro-1,4-anthrachinon,
2-Brom-3-(3-brom-5-tert-butyl-4-hydroxyphenyl)-5,5,8,8-tetramethyl-5,6,7,8-tetrahydro-1,4-anthrachinon,
2-Brom-3-(3,5-dibrom-4-hydroxyphenyl)-5,5,8,8-tetramethyl-5,6,7,8-tetrahydro-1,4-anthrachinon,
2-Brom-3-(3-brom-5-tert-butyl-4-hydroxyphenyl)-3-hydroxy-5,5,8,8-tetramethyl-5,6,7,8-tetrahydro-1,4-anthrachinon,
3-Brom-2-(3,5-di-tert-butyl-4-hydroxyphenyl)-1,4-anthrachinon,
2-(3,5-Di-tert-butyl-4-hydroxyphenyl)-3-methoxy-1,4-anthrachinon,
2-(3,5-Di-tert-butyl-4-hydroxyphenyl)-3-hydroxy-1,4-anthrachinon,
5,5,8,8-Tetramethyl-5,6,7,8-tetrahydronaphthalin-1,3-diol,
3-Methoxy-5,5,8,8-tetramethyl-5,6,7,8-tetrahydronaphthalin-1-ol,
4-(3-Chlor-5,5,8,8-tetramethyl-1,4-dioxo-1,4,5,6,7,8-hexahydroanthracen-2-yl)-benzoesäure,
Methyl-4-(3-chlor-5,5,8,8-tetramethyl-1,4-dioxo-1,4,5,6,7,8-hexahydroanthracen-2-yl)-benzoat,
4-(3-Hydroxy-1,4-dioxo-1,4-dihydronaphthalin-2-yl)-benzoesäure,
Methyl-(3-methoxy-1,4-dioxo-1,4-dihydronaphthalin-2-yl)-benzoesäure,
4-(3-Hydroxy-5,5,8,8-tetramethyl-1,4-dioxo-1,4,5,6,7,8-hexahydroanthracen-2-yl)-benzoesäure, Methyl-4-(3-hydroxy-1,4-dioxo-1,4-dihydronaphthalin-2-yl-azo)-benzoat,
4-(3-Hydroxy-5,5,8,8-tetramethyl-1,4-dioxo-1,4,5,6,7,8-hexahydroanthracen-2-yl-azo)-benzoesäure, 3-(3,5-Di-tert-butyl-4-oxocyclohexa-2,5-dienyliden)-5,5,8,8-tetramethyl-5,6,7,8-tetrahydrocyclopenta[b]naphthalin-1,2-dion,
3-(3,5-Di-tert-butyl-4-oxocyclohexa-2,5-dienyliden)-5,5,8,8-tetramethyl-5,6,7,8-tetrahydroanthracen-3H-1,2,4-trion,
2-(3,5-Di-tert-butyl-4-hydroxyphenyl)-3-methoxy-5,8-dimethyl-1,4-naphthochinon,
2-(3,5-Di-tert-butyl-4-hydroxyphenyl)-3-methoxy-6,7-dimethyl-1,4-naphthochinon,
2-(3,5-Di-tert-butyl-4-hydroxyphenyl)-3-methoxy-5-methyl-1,4-naphthochinon,
2-(3,5-Di-tert-butyl-4-hydroxyphenyl)-2-methoxy-5-methyl-1,4-naphthochinon,
2-(3,5-Di-tert-butyl-4-hydroxyphenyl)-3-methoxy-6-methyl-1,4-naphthochinon,
3-(3,5-Di-tert-butyl-4-hydroxyphenyl)-2-methoxy-6-methyl-1,4-naphthochinon,
2-(3,5-Di-tert-butyl-4-hydroxyphenyl)-3-methoxy-5,6-dimethyl-1,4-naphthochinon,
3-(3,5-Di-tert-butyl-4-hydroxyphenyl)-2-methoxy-5,6-dimethyl-1,4-naphthochinon,
2-(3,5-Di-tert-butyl-4-hydroxyphenyl)-3-methoxy-5,7-dimethyl-1,4-naphthochinon,
3-(3,5-Di-tert-butyl-4-hydroxyphenyl)-2-methoxy-5,7-dimethyl-1,4-naphthochinon,
2-(3,5-Di-tert-butyl-4-hydroxyphenyl)-3-ethylthio-5-methyl-1,4-naphthochinon,
2-(3,5-Di-tert-butyl-4-hydroxyphenyl)-3-ethylthio-6-methyl-1,4-naphthochinon,
2-(3,5-Di-tert-butyl-4-hydroxyphenyl)-3-hydroxy-5,8-dimethyl-1,4-naphthochinon,
2-(3,5-Di-tert-butyl-4-hydroxyphenyl)-3-hydroxy-6,7-dimethyl-1,4-naphthochinon,
2-(3,5-Di-tert-butyl-4-hydroxyphenyl)-3-hydroxy-5-methyl-1,4-naphthochinon,
3-(3,5-Di-tert-butyl-4-hydroxyphenyl)-2-hydroxy-5-methyl-1,4-naphthochinon,
2-(3,5-Di-tert-butyl-4-hydroxyphenyl)-3-hydroxy-6-methyl-1,4-naphthochinon,
3-(3,5-Di-tert-butyl-4-hydroxyphenyl)-2-hydroxy-6-methyl-1,4-naphthochinon,
2-(3,5-Di-tert-butyl-4-hydroxyphenyl)-3-hydroxy-5,6-dimethyl-1,4-naphthochinon,
2-(3-Brom-5-tert-butyl-4-hydroxyphenyl)-3-hydroxy-5,6-dimethyl-1,4-naphthochinon,
3-(3,5-Di-tert-butyl-4-hydroxyphenyl)-2-hydroxy-5,6-dimethyl-1,4-naphthochinon,
2-(3,5-Di-tert-butyl-4-hydroxyphenyl)-3-hydroxy-5,7-dimethyl-1,4-naphthochinon,
3-(3,5-Di-tert-butyl-4-hydroxyphenyl)-2-hydroxy-5,7-dimethyl-1,4-naphthochinon.

4. Verfahren nach einem der voranstehenden Ansprüche, **dadurch gekennzeichnet, dass** man die genomische DNA-Probe vor der Behandlung thermisch denaturiert.

5. Verfahren nach Anspruch 1 **dadurch gekennzeichnet, dass** man den Schritt c) in zwei Teilschritten wie folgt durchführt:
a) eine PCR Präamplifikation mit mindestens einem Primerpaar unterschiedlicher Sequenz, die an eine nach Anspruch 1 vorbehandelte DNA-Probe unspezifisch hybridisieren und daher im PCR Schritt mehr als ein Amplifikat ergeben;
b) eine PCR Amplifikation des in der Präamplifikation gebildeten Produkts mit Primern unterschiedlicher Sequenz, die jeweils zu einem Abschnitt der nach Anspruch 1 vorbehandelten DNA-Probe [(+)-Strang oder (-)-Strang] identisch oder komplementär sind und die zu amplifizierende DNA spezifisch hybridisieren.

6. Verfahren nach einem der vorangehenden Ansprüche, **dadurch gekennzeichnet, dass** man die Amplifikation von mehreren DNA-Abschnitten in einem Reaktionsgefäß durchführt.

7. Verfahren nach einem der vorangehenden Ansprüche, **dadurch gekennzeichnet, dass** man für die Polymerasereaktion eine hitzebeständige DNA-Polymerase verwendet.

8. Verfahren nach einem der vorangehenden Ansprüche, **dadurch gekennzeichnet, dass** man für die Detektion der vorbehandelten DNA die PCR-Produkte auf einen Oligonukleotid Array hybridisiert und man anschließend die folgenden Teilschritte ausführt:
a) die amplifizierte genomische DNA wird an mindestens ein Oligonukleotid unter Ausbildung einer Duplex hybridisiert, wobei besagte hybridisierte Oligonukleotide mit ihrem 3'-Ende unmittelbar oder im Abstand von bis zu 10 Basen an die Positionen angrenzen, die hinsichtlich ihrer Methylierung in der genomischen DNA-Probe zu untersuchen sind;
(b) man das Oligonukleotid mit bekannter Sequenz von n Nukleotiden mittels einer Polymerase mindestens um ein Nukleotid verlängert, wobei das Nukleotid eine nachweisbare Markierung trägt und die Verlängerung vom Methylierungsstatus des jeweiligen Cytosins in der genomischen DNA-Probe abhängt.

9. Verfahren nach einem der Ansprüche 1 bis 7 **dadurch gekennzeichnet, dass** man für die Detektion der vorbehandelten DNA die PCR-Produkte auf einen Oligonukleotid Array hybridisiert und man anschließend die folgenden Teilschritte ausgeführt:
(a) man hybridisiert einen Satz von Oligonukleotiden an die amplifizierte genomische DNA unter Ausbildung einer Duplex, wobei dieser Satz von Oligonukleotiden aus zwei verschiedenen Spezies besteht und wobei die hybridisierten Oligonukleotide der ersten Spezies mit ihrem 3'-Ende unmittelbar oder im Abstand von bis zu 10 Basen an die Positionen angrenzen, die hinsichtlich ihrer Methylierung in der genomischen DNA-Probe zu untersuchen sind und wobei das zweite Oligonukleotid der zweiten Spezies an eine zweite Region des Zielmoleküls hybridisiert, so dass das 5'-Ende des Oligonukleotids der zweiten Spezies durch eine Lücke von der Größe eines Einzelnukleotides oder bis zu 10 Nukleotiden vom 3'-Ende des hybridisierten Oligonukleotids der ersten Spezies an der Stelle der besagten ausgewählten Position getrennt ist;
(b) man das Oligonukleotid der ersten Spezies mit bekannter Sequenz von n Nukleotiden mittels einer Polymerase um höchstens die Anzahl von Nukleotiden verlängert, die zwischen dem 3'-Ende des Oligonukleotids der 1. Spezies und dem 5'-Ende des Oligonukleotids der 2. Spezies liegen, wobei die Verlängerung vom Methylierungsstatus des jeweiligen Cytosins in der genomischen DNA-Probe abhängt;
(c) man inkubiert die Oligonukleotide in Gegenwart einer Ligase, wobei das angrenzende, durch die Polymerasereaktion verlängerte Oligonukleotid der ersten Spezies und das Oligonukleotid der zweiten Spezies verbunden werden und man dadurch ein Ligationsprodukt erhält, sofern im vorangehenden Schritt eine Verlängerung des Oligonukleotids der ersten Spezies derart erfolgte, dass nun das 3'-Ende mit vorhandener 3'-Hydroxyfunktion des verlängerten Oligonukleotids unmittelbar an das 5'-Ende des Oligonukleotids der zweiten Spezies angrenzt.

10. Verfahren nach Anspruch 9, **dadurch gekennzeichnet, dass** die verwendeten Oligonukleotide der ersten Spezies und/oder die verwendeten Oligonukleotide der zweiten Spezies entweder nur die Basen T, A und C oder aber die Basen T, A und G enthalten.

11. Verfahren nach einem der Ansprüche 1 bis 7, **dadurch gekennzeichnet, dass** man für die Detektion der vorbehandelten DNA die PCR-Produkte auf einen Oligonukleotid Array hybridisiert und man anschließend die folgenden Teilschritte ausführt:
(a) man hybridisiert die amplifizierte genomische DNA an mindestens ein Oligonukleotid mit bekannter Sequenz von n Nukleotiden unter Ausbildung einer Duplex, wobei besagte hybridisierte Oligonukleotide mit ihrem 3'-Ende teilweise oder vollständig an die Positionen hybridisieren, die hinsichtlich ihrer Methylierung in der genomischen DNA-Probe zu untersuchen sind;
(b) man das Oligonukleotid, sofern es mit seinem 3'-Terminus zuvor ohne Basenfehlpaarungen an die zu untersuchenden Position hybridisierte, mittels einer Polymerase mindestens um ein Nukleotid verlängert, wobei mindestens ein Nukleotid eine nachweisbare Markierung trägt und die Verlängerung vom Methylierungsstatus des jeweiligen Cytosins in der genomischen DNA-Probe abhängt.

12. Verfahren nach einem der voranstehenden Ansprüche, **dadurch gekennzeichnet, dass** man die PCR-Produkte und/oder Verlängerungsprodukte und/oder Ligationsprodukte für die Detektion mit einer nachweisbaren Markierung versieht.

13. Verfahren nach einem der voranstehenden Ansprüche, **dadurch gekennzeichnet, dass** die Markierungen Fluoreszenzmarkierungen sind.

14. Verfahren nach einem der voranstehenden Ansprüche, **dadurch gekennzeichnet, dass** die Markierungen Radionuklide sind.

15. Verfahren nach einem der Ansprüche 1 bis 12, **dadurch gekennzeichnet, dass** die Markierungen der Nukleotide ablösbare Massenmarkierungen sind, die in einem Massenspektrometer nachweisbar sind.

16. Verfahren nach einem der Ansprüche 1 bis 12, **dadurch gekennzeichnet, dass** man die PCR-Produkte und/oder Verlängerungsprodukte und/oder Ligationsprodukte insgesamt im Massenspektrometer nachweist und somit durch ihre Masse eindeutig charakterisiert sind.

17. Verfahren nach einem der Ansprüche 1 bis 12, **dadurch gekennzeichnet, dass** man jeweils ein Fragment der PCR-Produkte und/oder Verlängerungsprodukte und/oder Ligationsprodukte im Massenspektrometer nachweist.

18. Verfahren nach Anspruch 17, **dadurch gekennzeichnet, dass** man das Fragment des PCR-Produkts und/oder Verlängerungsprodukts und/oder Ligationsprodukts durch Verdau mit einer oder mehrerer Exo- oder Endonukleasen erzeugt.

19. Verfahren nach Anspruch 17 und 18, **dadurch gekennzeichnet, dass** man zur besseren Detektierbarkeit im Massenspektrometer die erzeugten Fragmente mit einer einzelnen positiven oder negativen Nettoladung versieht.

20. Verfahren gemäß einem der voranstehenden Ansprüche, **dadurch gekennzeichnet, dass** man die PCR-Produkte und/oder Verlängerungsprodukte und/oder Ligationsprodukte mittels Matrix assistierter Laser Desorptions/Ionisations Massenspektrometrie (MALDI-TOF) oder mittels Elektrospray Massenspektrometrie (ESI) detektiert und visualisiert.

21. Verfahren nach einem der voranstehenden Ansprüche, wobei man die genomische DNA aus einer DNA-Probe erhält, wobei Quellen für DNA z. B. Zelllinien, Blut, Sputum, Stuhl, Urin, Gehirn-Rückenmarks-Flüssigkeit, in Paraffin eingebettetes Gewebe, beispielsweise Gewebe von Augen, Darm, Niere, Hirn, Herz, Prostata, Lunge, Brust oder Leber, histologische Objektträger und alle möglichen Kombinationen hiervon umfassen.

22. Verwendung eines Verfahrens nach einem der voranstehenden Ansprüche zur Diagnose und/oder Prognose nachteiliger Ereignisse für Patienten oder Individuen, wobei diese nachteiligen Ereignisse mindestens einer der folgenden Kategorien angehören: unerwünschte Arzneimittelwirkungen; Krebserkrankungen; CNS-Fehlfunktionen, Schäden oder Krankheit; Aggressionssymptome oder Verhaltensstörungen; klinische, psychologische und soziale Konsequenzen von Gehirnschädigungen; psychotische Störungen und Persönlichkeitsstörungen; Demenz und/oder assoziierte Syndrome; kardiovaskuläre Krankheit, Fehlfunktion und Schädigung; Fehlfunktion, Schädigung oder Krankheit des gastrointestinalen Traktes; Fehlfunktion, Schädigung oder Krankheit des Atmungssystems; Verletzung, Entzündung, Infektion, Immunität und/oder Rekonvaleszenz; Fehlfunktion, Schädigung oder Krankheit des Körpers als Abweichung im Entwicklungsprozess; Fehlfunktion, Schädigung oder Krankheit der Haut, der Muskeln, des Bindegewebes oder der Knochen; endokrine und metabolische Fehlfunktion, Schädigung oder Krankheit; Kopfschmerzen oder sexuelle Fehlfunktion.

23. Verwendung eines Verfahrens nach einem der voranstehenden Ansprüche zur Unterscheidung von Zelltypen oder Geweben oder zur Untersuchung der Zelldifferenzierung.

24. Kit, bestehend aus einem Bisulfit enthaltenen Reagenz, denaturierenden Reagenzien oder Lösungsmitteln, sowie Radikalfängern und Primern zur Herstellung der Amplifikate, sowie eine Anleitung zur Durchführung eines Assays nach einem der Ansprüche 1 bis 21.


SEQUENZPROTOKOLL

<110> Epigenomics AG

<120> Verfahren zum Nachweis von Cytosin-Methylierungen

<130> E01/1204/WO

<140>
<141>

<160> 2

<170> PatentIn Ver. 2.1

<210> 1
<211> 28
<212> DNA
<213> Künstliche Sequenz

<220>
<223> Beschreibung der künstlichen Sequenz: Primer

<400> 1
agggagtttt ttttagggaa tagaggga                                    28

<210> 2
<211> 28
<212> DNA
<213> Künstliche Sequenz

<220>
<223> Beschreibung der künstlichen Sequenz:Primer

<400> 2
taatcccaaa acctctccac tacaacaa                                    28